(19)

**Europäisches Patentamt**

**European Patent Office**

**Office européen des brevets**

(11) **EP 4 291 125 B1**

(12) **EUROPEAN PATENT SPECIFICATION**

(45) Date of publication and mention
of the grant of the patent:
**04.02.2026 Bulletin 2026/06**

(21) Application number: **22709859.7**

(22) Date of filing: **08.02.2022**

(51) International Patent Classification (IPC):
***A61B 18/26*** (2006.01)

(52) Cooperative Patent Classification (CPC):
**A61B 18/26;** A61B 18/042; A61B 2018/0022;
A61B 2018/2035; A61B 2018/2205;
A61B 2018/2211; A61B 2018/2244; A61B 2018/263;
A61B 2018/266

(86) International application number:
**PCT/US2022/015577**

(87) International publication number:
**WO 2022/173719 (18.08.2022 Gazette 2022/33)**

(54) **OPTICAL ASSEMBLIES TO IMPROVE ENERGY COUPLING TO PRESSURE WAVE GENERATOR OF AN INTRAVASCULAR LITHOTRIPSY DEVICE**

OPTISCHE ANORDNUNGEN ZUR VERBESSERUNG DER ENERGIEKOPPLUNG MIT EINEM DRUCKWELLENGENERATOR EINER INTRAVASKULÄREN LITHOTRIPSIEVORRICHTUNG

ENSEMBLES OPTIQUES POUR AMÉLIORER LE COUPLAGE ÉNERGÉTIQUE À UN GÉNÉRATEUR D'ONDES DE PRESSION D'UN DISPOSITIF DE LITHOTRITIE INTRAVASCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.02.2021 US 202163148133 P**
**07.02.2022 US 202217666172**

(43) Date of publication of application:
**20.12.2023 Bulletin 2023/51**

(60) Divisional application:
**25221167.7**

(73) Proprietor: **Bolt Medical, Inc.**
**Carlsbad, CA 92008 (US)**

(72) Inventors:
• **COOK, Christopher, A.**
**Laguna Niguel, CA 92677 (US)**
• **SCHULTHEIS, Eric**
**San Clemente, CA 92673 (US)**
• **MOSSAYEBI, Mina**
**Irvine, CA 92602 (US)**
• **LIU, George**
**Irvine, CA 92620 (US)**
• **FANG, Itzhak**
**Irvine, CA 92614 (US)**
• **JOHNSON, Jennet**
**Fallbrook, CA 92028 (US)**
• **SHAR, Theresa**
**San Diego, CA 92126 (US)**

(74) Representative: **Vossius & Partner**
**Patentanwälte Rechtsanwälte mbB**
**P.O. Box 86 07 67**
**81634 München (DE)**

(56) References cited:
**US-A1- 2016 184 023     US-A1- 2020 129 195**
**US-A1- 2020 397 230**

## Description

### BACKGROUND

Vascular lesions within and adjacent to vessels in the body can be associated with an increased risk for major adverse events, such as myocardial infarction, embolism, deep vein thrombosis, stroke, and the like. Severe vascular lesions can be difficult to treat and achieve patency for a physician in a clinical setting.

Vascular lesions may be treated using interventions such as drug therapy, balloon angioplasty, atherectomy, stent placement, vascular graft bypass, to name a few. Such interventions may not always be ideal or may require subsequent treatment to address the lesion.

Using optical fiber delivery of laser pulses to generate high-pressure impulses on the lesions is one way to attempt to treat the lesions. The creation of plasma via optical breakdown of an aqueous solution typically requires a significant amount of energy in a short amount of time upon which it is converted into a therapeutic bubble and/or a therapeutic pressure wave. In this context, reference is made to document US2020/0397230 A1.

### SUMMARY

The present invention is directed toward a catheter system for treating a treatment site within or adjacent to a vessel wall or a heart valve. The catheter system includes an inflatable balloon, an optical fiber, and an energy source. In certain embodiments, the optical fiber has (i) a fiber proximal end and (ii) a fiber distal end positioned within the inflatable balloon. The optical fiber can be configured to receive an energy pulse so that the optical fiber emits light energy in a direction away from the optical fiber to generate a plasma pulse within the inflatable balloon, the optical fiber being tapered from the fiber proximal end to the fiber distal end. The energy source can be in optical communication with the fiber proximal end of the optical fiber.

In various embodiments, the optical fiber can be tapered using a fusion splicer.

In certain embodiments, the optical fiber can be spliced using a core matched fiber.

In some embodiments, the optical fiber can be split into a first fiber member and a second fiber member.

In various embodiments, the optical fiber can be split so that only the second fiber member can be positioned within the inflatable balloon.

In certain embodiments, the second fiber member can include a plurality of second fiber legs.

In some embodiments, the second fiber member can include two second fiber legs.

In various embodiments, the second fiber member can include three second fiber legs.

In certain embodiments, the first fiber member can be fused to the second fiber member in a fused region.

In some embodiments, the first fiber member and the second fiber member are formed as a unitary structure.

In various embodiments, the first fiber member and the second fiber member are integrally formed with one another.

In certain embodiments, the first fiber member and the second fiber member are continuously formed as a single structure.

In some embodiments, the first fiber member can have a first fiber member distal region that is outside the inflatable balloon.

In various embodiments, the second fiber member can have a second fiber member proximal region that is outside the inflatable balloon.

In certain embodiments, the first fiber member distal region can be fused to the second fiber member proximal region in the fused region.

In some embodiments, the energy source can include a laser.

In various embodiments, the catheter system can include a ferrule that encircles the fused region.

In certain embodiments, the catheter system can include a ferrule that encircles a first fiber proximal end of the first optical fiber and the first fiber distal end of the second fiber.

In some embodiments, the catheter system can include a plurality of ferrules that encircle portions of the first fiber member and the second fiber member.

In various embodiments, the ferrule can have an outer diameter of 1.25mm LC ferrules.

In certain embodiments, the ferrule can have an outer diameter of 2.5mm SC ferrules.

In some embodiments, the ferrule can be formed by one of a plastic and a metal.

In various embodiments, the ferrule can be configured for only one optical fiber.

In certain embodiments, the ferrule can be configured for a plurality of fiber members.

In some embodiments, the ferrule can be one of a multi-fiber MT ferrule and an MTP ferrule.

In various embodiments, the optical fiber can be cleaved at a first tapered portion of the first fiber member.

In certain embodiments, the optical fiber can be cleaved by one of a laser cleaver and a mechanical cleaver.

In some embodiments, the first fiber member can have a first cleaved portion that can be cleaved a second time to form a curved ball surface.

In various embodiments, the optical fiber can be tapered with rotation.

In certain embodiments, the optical fiber can be tapered without rotation.

In some embodiments, the ferrule can include at least one of a tapered fiber, a spliced joint, and a bare fiber.

In various embodiments, at least one of the tapered fiber, the spliced joint, and the bare fiber are encircled inside the ferrule with an epoxy.

In certain embodiments, the epoxy can be ND353 encircled for fiber connector polishing.

In some embodiments, the epoxy can be LOCTITE® 4310 UV adhesive.

In various embodiments, the optical fiber can be tapered from a diameter of 200 $\mu$m to a diameter of 105 $\mu$m.

In certain embodiments, the optical fiber can be configured to split a light energy traveling through the first fiber member into the second fiber member.

In some embodiments, the first fiber member can include an endcap.

In various embodiments, the ferrule substantially surrounds the first fiber member.

In certain embodiments, the optical fiber can be tapered so that it can have a greater diameter on the fiber proximal end and a lesser diameter on the fiber distal end.

The present invention may be used in a method for treating a treatment site within or adjacent to a vessel wall or heart valve. The method can include the steps of positioning an optical fiber having (i) a fiber proximal end and (ii) a fiber distal end positioned within the inflatable balloon, the optical fiber being configured to receive an energy pulse so that the optical fiber emits light energy in a direction away from the optical fiber to generate a plasma pulse within the inflatable balloon, and coupling an energy source with the fiber proximal end of the optical fiber so that the energy source is in optical communication with the optical fiber.

The method can include the step of tapering the optical fiber using a fusion splicer.

Furthermore, the method can include the step of splicing the optical fiber using a core matched fiber.

Furthermore, the method can include the step of encircling the fused region using a ferrule.

Furthermore, the method can include the step of encircling a first fiber distal end of the first fiber member and the second fiber proximal end of the second fiber member using a ferrule.

Furthermore, the method can include the step of encircling portions of the first fiber member and the second fiber member using a plurality of ferrules.

The present invention is also directed toward a catheter system that includes an inflatable balloon, an optical fiber, an energy source, and a fused region. The optical fiber can have (i) a fiber proximal end and (ii) a fiber distal end positioned within the inflatable balloon. The optical fiber can be configured to receive an energy pulse so that the optical fiber emits light energy in a direction away from the optical fiber to generate a plasma pulse within the inflatable balloon. The optical fiber can include a first fiber member and a second fiber member. The energy source can be in optical communication with the fiber proximal end of the optical fiber. The fused region can be located where the first fiber member is fused to the second fiber member.

The present invention is further directed toward a catheter system that includes an inflatable balloon, an optical fiber, an energy source, and a fused region. The optical fiber can have (i) a fiber proximal end and (ii) a fiber distal end positioned within the inflatable balloon. The optical fiber can be configured to receive an energy pulse so that the optical fiber emits light energy in a direction away from the optical fiber to generate a plasma pulse within the inflatable balloon. The optical fiber can have a first fiber member and a second fiber member including a plurality of second fiber member legs. The energy source can be in optical communication with the fiber proximal end of the optical fiber. The fused region can be located where the first fiber member is fused to the second fiber member. The fused region can be configured to split the light energy traveling through the first fiber member into the second fiber member so that the light energy is split between the plurality of the second fiber member legs.

The present invention is also directed toward a catheter system that includes an inflatable balloon, an optical fiber, an energy source, a fused region, and a capillary. The optical fiber can have (i) a fiber proximal end and (ii) a fiber distal end positioned within the inflatable balloon. The optical fiber can be configured to receive an energy pulse so that the optical fiber emits light energy in a direction away from the optical fiber to generate a plasma pulse within the inflatable balloon. The optical fiber can have a first fiber member and a second fiber member including a plurality of second fiber member legs. The energy source can be in optical communication with the fiber proximal end of the optical fiber. The fused region can be located where the first fiber member is fused to the second fiber member. The fused region can be configured to split the light energy traveling through the first fiber member into the second fiber member so that the light energy is split between the plurality of the second fiber member legs. The capillary can substantially surround the fused region.

The present invention is further directed toward a catheter system that includes an inflatable balloon, an optical fiber, a fused region, and a ferrule. The optical fiber can have (i) a fiber proximal end and (ii) a fiber distal end positioned within the inflatable balloon. The optical fiber can be configured to receive an energy pulse so that the optical fiber emits light energy in a direction away from the optical fiber to generate a plasma pulse within the inflatable balloon. The optical fiber can include an endcap and a first fiber distal end positioned within the inflatable balloon. The optical fiber can have a first fiber member and a second fiber member. The fused region can be located where the first fiber member is fused to the second fiber member. The ferrule can substantially surround the fused region.

The present invention is also directed toward a catheter system that includes an inflatable balloon, an optical fiber, an energy source, a fused region, and a ferrule. The optical fiber can have (i) a fiber proximal end, and (ii) a fiber distal end positioned within the inflatable balloon. The optical fiber can be configured to receive an energy pulse so that the optical fiber emits light energy in a direction away from the optical fiber to generate a plasma pulse within the inflatable balloon. The optical fiber can include a first fiber member and a second fiber member. The energy source can be in optical communication with the fiber proximal end of the optical fiber. The fused region can be located where the first fiber member is fused to the second fiber member. The ferrule can substantially surround the first fiber member and the fused region.

The present invention is further directed toward a catheter system that includes an inflatable balloon and an optical fiber. The optical fiber can have (i) a fiber proximal end, and (ii) a fiber distal end positioned within the inflatable balloon. The optical fiber can be configured to receive an energy pulse so that the optical fiber emits light energy in a direction toward the fiber distal end to generate a plasma pulse within the inflatable balloon. The optical fiber can be tapered so that it can have a greater diameter on the fiber proximal end and a lesser diameter on the fiber distal end.

The present invention is also directed toward a catheter system that includes an inflatable balloon and an optical fiber. The optical fiber can have (i) a fiber proximal end, and (ii) a fiber distal end positioned within the inflatable balloon. The optical fiber can be configured to receive an energy pulse so that the optical fiber emits light energy in a direction away from the optical fiber to generate a plasma pulse within the inflatable balloon. The optical fiber can be tapered from the fiber proximal end to the fiber distal end. The energy source can be in optical communication with the fiber proximal end of the optical fiber.

The present invention is further directed toward a catheter system that includes an inflatable balloon and an optical fiber. The optical fiber can have (i) a fiber proximal end, and (ii) a fiber distal end positioned within the inflatable balloon. The optical fiber can be configured to receive an energy pulse so that the optical fiber emits light energy in a direction away from the optical fiber to generate a plasma pulse within the inflatable balloon. The optical fiber can be split into a plurality of fiber members positioned within the inflatable balloon. The energy source can be in optical communication with the fiber proximal end of the optical fiber.

In various embodiments, the optical fiber can be tapered from a diameter of $220\mu$m to a diameter of $100\mu$m.

In some embodiments, the optical fiber can be tapered from (i) an initial diameter to (ii) a final diameter having a diameter of 90% to 10% of the initial diameter.

In certain embodiments, the catheter system can include a third fiber member and a second fused region, the third fiber member being fused to the second fiber member in the second fused region, the second fiber member further including a second tapered portion.

The present invention is also directed toward a catheter system for treating a treatment site within or adjacent to a vessel wall or heart valve. The catheter system can include an inflatable balloon, an optical fiber, an energy source, a first tapered portion, a second tapered portion, a first fused region, a second fused region, and a ferrule. The optical fiber can have (i) a fiber proximal end, and (ii) a fiber distal end positioned within the inflatable balloon, the optical fiber being configured to receive an energy pulse so that the optical fiber emits light energy in a direction away from the optical fiber to generate a plasma pulse within the inflatable balloon, the optical fiber having a first fiber member, a second fiber member, and a third fiber member. The energy source can be optical communication with the fiber proximal end of the optical fiber. The first tapered portion can be located on the first fiber member. The second tapered portion can be located on the second fiber member. The first fused region where the first fiber member is fused to the second fiber member. The second fused region can be where the second fiber member is fused to the third fiber member. The ferrule can substantially surround the first fiber member and the first fused region.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims.

## BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:

Figure 1 is a schematic cross-sectional view of one embodiment of a catheter system having features of the present invention;

Figure 2 is a schematic cross-sectional view of one embodiment of a portion of the catheter system including an optical fiber, coupling optics, and an energy source;

Figure 3A is a schematic cross-sectional view of one embodiment of a portion of the catheter system including an embodiment of the optical fiber;

Figure 3B is a schematic cross-sectional view of another embodiment of another optical fiber for use within a catheter system;

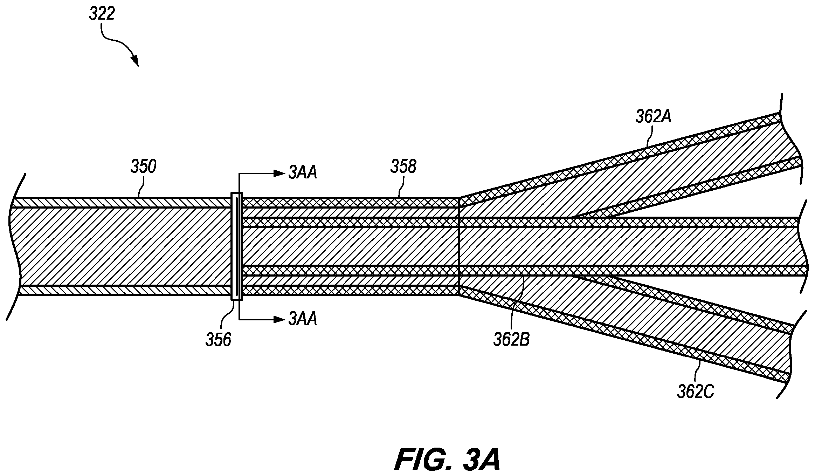

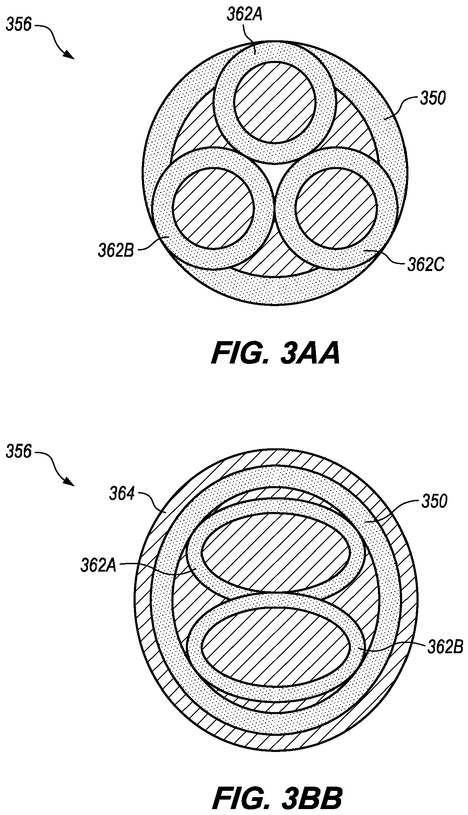

Figure 3AA is a cross-sectional view of the optical fiber taken on line 3AA-3AA in Figure 3A;

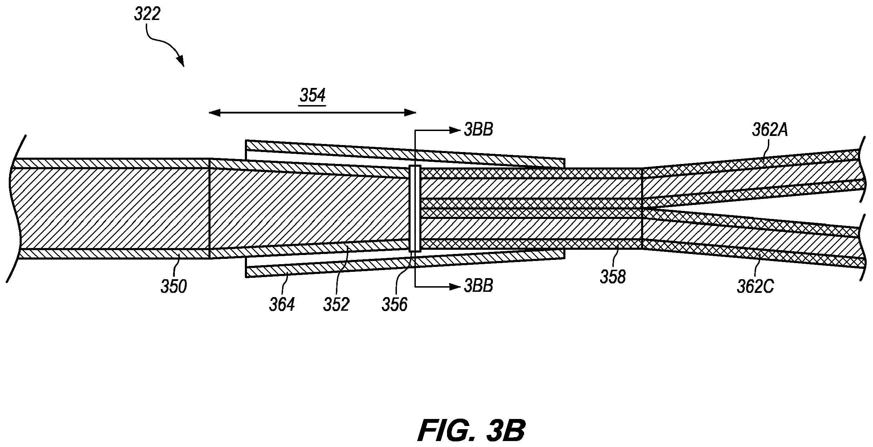

Figure 3BB is a cross-sectional view of the optical fiber taken on line 3BB-3BB in Figure 3B;

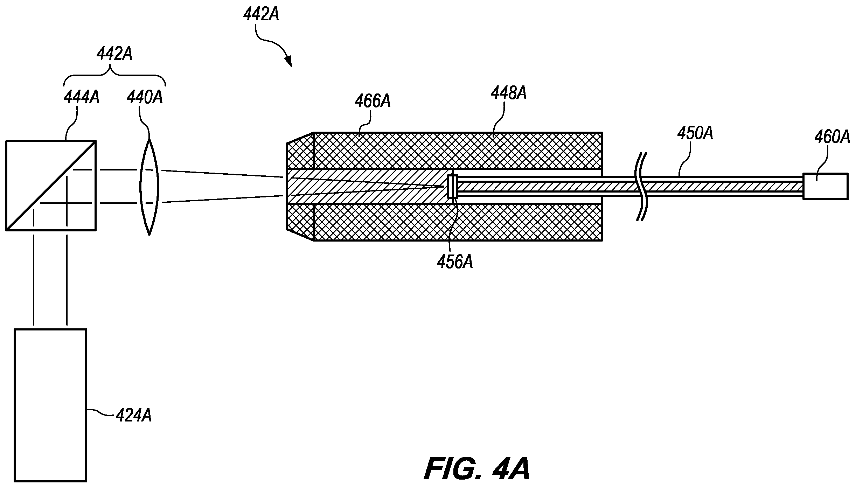

Figure 4A is a schematic cross-sectional view of another embodiment of the catheter system including an embodiment of the optical fiber, the coupling optics, and the energy source; and

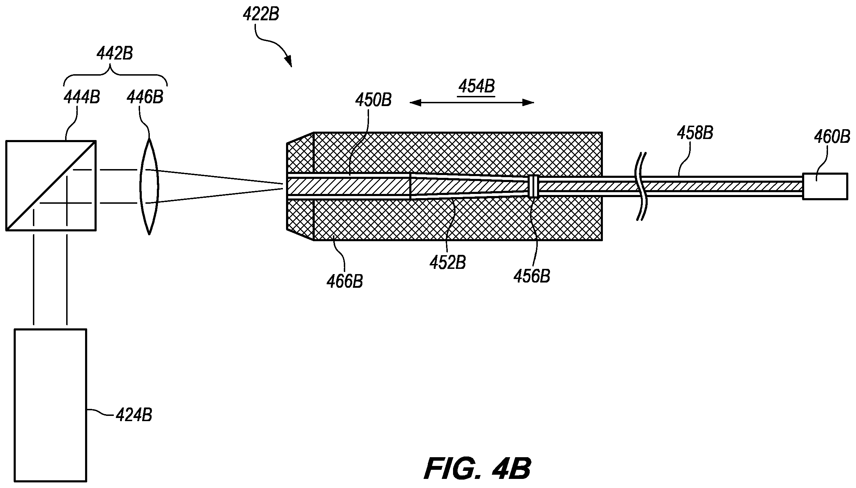

Figure 4B is a schematic cross-sectional view of another embodiment of the catheter system including another embodiment of the optical fiber, the coupling optics, and the energy source.

While embodiments are susceptible to various modifications and alternative forms, specifics thereof have been shown by way of example and drawings, and will be described in detail. It should be understood, however, that the scope herein is not limited to the particular aspects described.

## DESCRIPTION

Treatment of vascular lesions can reduce major adverse events or death in affected subjects. A major adverse event is one that can occur anywhere within the body due to the presence of a vascular lesion. Major adverse events can include, but are not limited to major adverse cardiac events, major adverse events in the peripheral or central vasculature, major adverse events in the brain, major adverse events in the musculature, or major adverse events in any of the internal organs.

As used herein, the treatment site can include a vascular lesion such as a calcified vascular lesion or a fibrous vascular lesion (hereinafter sometimes referred to simply as a "lesion" or "treatment site"), typically found in a blood vessel and/or a heart valve. Plasma formation can initiate a pressure wave and can initiate the rapid formation of one or more bubbles that can rapidly expand to a maximum size and then dissipate through a cavitation event that can also launch a pressure wave upon collapse. The rapid expansion of the plasma-induced bubbles can generate one or more pressure waves within a balloon fluid and thereby impart pressure waves upon the treatment site. The pressure waves can transfer mechanical energy through an incompressible balloon fluid to a treatment site to impart a fracture force on the lesion. Without wishing to be bound by any particular theory, it is believed that the rapid change in balloon fluid momentum upon a balloon wall of the inflatable balloon that is in contact with or positioned near the lesion is transferred to the lesion to induce fractures in the lesion.

Those of ordinary skill in the art will realize that the following detailed description of the present invention is illustrative only and is not intended to be in any way limiting. Other embodiments of the present invention will readily suggest themselves to such skilled persons having the benefit of this disclosure. Additionally, other methods of delivering energy to the lesion can be utilized, including, but not limited to, the electric current induced plasma generation. Reference will now be made in detail to implementations of the present invention as illustrated in the accompanying drawings.

In the interest of clarity, not all of the routine features of the implementations described herein are shown and described. It will, of course, be appreciated that in the development of any such actual implementation, numerous implementation-specific decisions must be made in order to achieve the developer's specific goals, such as compliance with application-related and business-related constraints, and that these specific goals will vary from one implementation to another and from one developer to another. Moreover, it is appreciated that such a development effort might be complex and time-consuming, but would nevertheless be a routine undertaking of engineering for those of ordinary skill in the art having the benefit of this disclosure.

As used herein, the terms "treatment site", "intravascular lesion" and "vascular lesion" are used interchangeably unless otherwise noted and can include lesions located at or near blood vessels or heart valves.

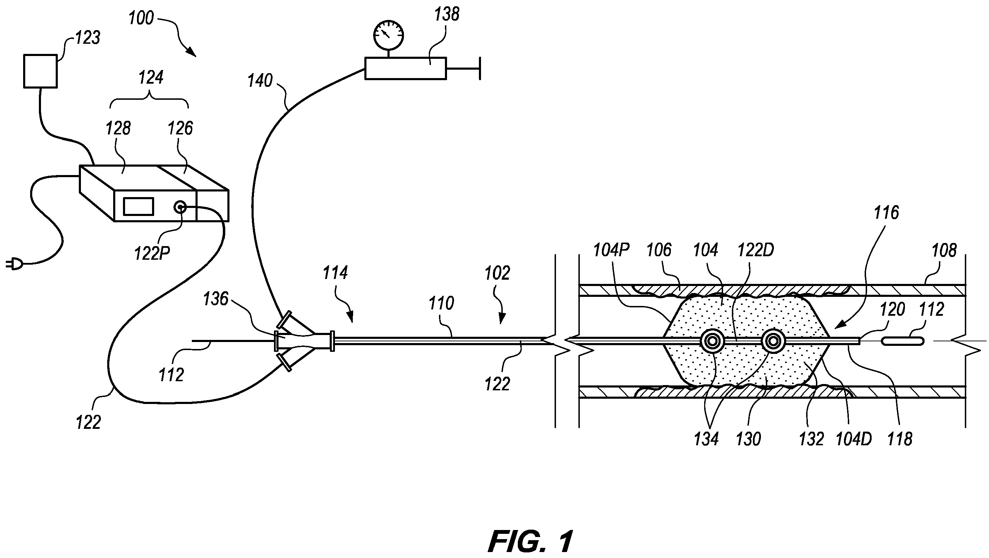

It is appreciated that the catheter systems herein can include many different forms and/or configurations other than those specifically shown and/or described herein. Referring now to Figure 1, a schematic cross-sectional view is shown of a catheter system in accordance with various embodiments herein. A catheter system 100 is suitable for imparting pressure to induce fractures in a vascular lesion within or adjacent to a vessel wall of a blood vessel and/or a heart valve. In the embodiment illustrated in Figure 1, the catheter system 100 can include one or more of a catheter 102, one or more optical fibers 122, a controller 123, an energy source 124, a manifold 136, a fluid pump 138, and a multiplexer (not shown).

The catheter 102 includes an inflatable balloon 104 (sometimes referred to herein as "balloon"). The catheter 102 is configured to move to a treatment site 106 within or adjacent to a blood vessel 108. The treatment site 106 can include a vascular lesion such as a calcified vascular lesion, for example. Additionally, or in the alternative, the treatment site 106 can include a vascular lesion such as a fibrous vascular lesion.

The catheter 102 can include the balloon 104, a catheter shaft 110, and a guidewire 112. The balloon can be coupled to the catheter shaft 110. The balloon can include a balloon proximal end 104P and a balloon distal end 104D. The catheter shaft 110 can extend between a shaft proximal end 114 and a shaft distal end 116. The catheter shaft 110 can include a guidewire lumen 118 which is configured to move over the guidewire 112. The catheter shaft 110 can also include an inflation lumen (not shown). In some embodiments, the catheter 102 can have a distal end opening 120 and can accommodate and be moved over and/or along the guidewire 112 so that the balloon 104 is positioned at or near the treatment site 106.

The balloon 104 can include a balloon wall 130. The balloon 104 can expand from a collapsed configuration suitable for advancing at least a portion of the catheter shaft 102 through a patient's vasculature to an expanded configuration suitable for anchoring the catheter 102 into position relative to the treatment site 106.

The catheter shaft 110 of the catheter 102 can encircle one or more optical fibers 122 (only one optical fiber 122 is illustrated in Figure 1 for clarity) in optical communication with the energy source 124. The optical fiber 122 can be at least partially disposed along and/or within the catheter shaft 110 and at least partially within the balloon 104. In some embodiments, the catheter shaft 110 can encircle multiple optical fibers 122 such as a second optical fiber, a third optical fiber, etc.

The optical fiber 122 can vary depending on the design requirements of the catheter system 100, and/or the energy source 124. It is understood that the optical fiber 122 can include additional systems, subsystems, components, and elements than those specifically shown and/or described herein. Additionally, or alternatively, the optical fiber 122 can omit one or more of the systems, subsystems, and elements that are specifically shown and/or described herein.

The optical fiber 122 has a fiber proximal end 122P that is positioned at or adjacent to the energy source 124, and a fiber distal end 122D that can be positioned within the inflatable balloon 104. The optical fiber 122 extends between the laser 124 and the balloon 104. The optical fiber 122 is in optical communication with the energy source 124. It is appreciated that the optical fiber 122 can be substituted with any suitable light carrier (the optical fiber 122 is a light carrier) configured to carry one or more sub-millisecond energy pulses and/or any suitable energy source.

The controller 123 can control the energy source 124 so that the energy source 124 can generate one or more energy pulses as provided in greater detail herein. The controller 123 may also perform any other relevant functions to control the operation of the catheter 102.

The energy source 124 of the catheter system 100 can be configured to provide one or more sub-millisecond energy pulses that are sent to and received by the optical fiber 122. The optical fiber 122 acts as a conduit for light energy that is generated by the energy pulse(s). In certain embodiments, the energy source 124 (also sometimes referred to herein as a "laser") can include a laser. In some such embodiments, the laser 124 can include one or more seed sources 126 and one or more amplifiers 128. Each amplifier 128 can be in optical communication with at least one of the seed sources 126. The seed source(s) 126 can each emit a relatively low-power seed pulse that is received and amplified by the amplifier 128. The amplifier 128 can increase the power of the seed pulse to generate the energy pulse. In one embodiment, the laser 124 can include one seed source 126 and one amplifier 128.

Alternatively, the laser 124 can include a plurality of seed sources 126 and one amplifier 128. Still alternatively, the laser 124 can include a plurality of seed sources 126 and a plurality of amplifiers 128. It is appreciated that the laser 124 can be substituted with any suitable energy source (the laser 124 is an energy source) configured to provide one or more sub-millisecond energy pulses that are sent to and received by the optical fiber 122.

The light energy that is generated by the energy pulse(s) is delivered by the optical fiber 122 to a location within the balloon 104. The light energy induces plasma formation in the form of a plasma pulse 134 that occurs in the balloon fluid 132 within the balloon 104. The plasma pulse 134 causes rapid bubble formation and imparts pressure waves upon the treatment site 106. Exemplary plasma pulses 134 are shown in Figure 1. The balloon fluid 132 can be a liquid or a gas. As provided in greater detail herein, the plasma-induced bubbles 134 are intentionally formed at some distance away from the optical fiber 122 so that the likelihood of damage to the optical fiber is decreased.

In various embodiments, the sub-millisecond pulses of light can be delivered to near the treatment site 106 at a frequency of from at least approximately 1 hertz (Hz) up to approximately 5000 Hz. In some embodiments, the sub-millisecond pulses of light can be delivered to near the treatment site 106 at a frequency from at least 30 Hz to 1000 Hz. In other embodiments, the sub-millisecond pulses of light can be delivered to near the treatment site 106 at a frequency from at least 10 Hz to 100 Hz. In yet other embodiments, the sub-millisecond pulses of light can be delivered to near the treatment site 106 at a frequency from at least 1 Hz to 30 Hz.

It is appreciated that the catheter system 100 herein can include any number of optical fibers 122 in optical communication with the laser 124 at the proximal portion 114 and with the balloon fluid 132 within the balloon 104 at the distal portion 116. For example, in some embodiments, the catheter system 100 herein can include 1-30 optical fibers 122. In some embodiments, the catheter system 100 herein can include greater than 30 optical fibers.

The manifold 136 can be positioned at or near the shaft proximal end 114. The manifold 136 can include one or more proximal end openings that can receive the one or more optical fibers, such as optical fiber 122, the guidewire 112, and/or an inflation conduit 140. The catheter system 100 can also include the fluid pump 138 that is configured to inflate the balloon 104 with the balloon fluid 132 and/or deflate the balloon 104 as needed.

The multiplexer (not shown) communicates with a single energy source into one or more of the optical channels in a tightly controlled manner. This approach can allow a single energy source to be channeled sequentially through a plurality of channels with a variable number.

As with all embodiments illustrated and described herein, various structures may be omitted from the figures for clarity and ease of understanding. Further, the figures may include certain structures that can be omitted without deviating from the intent and scope of the invention.

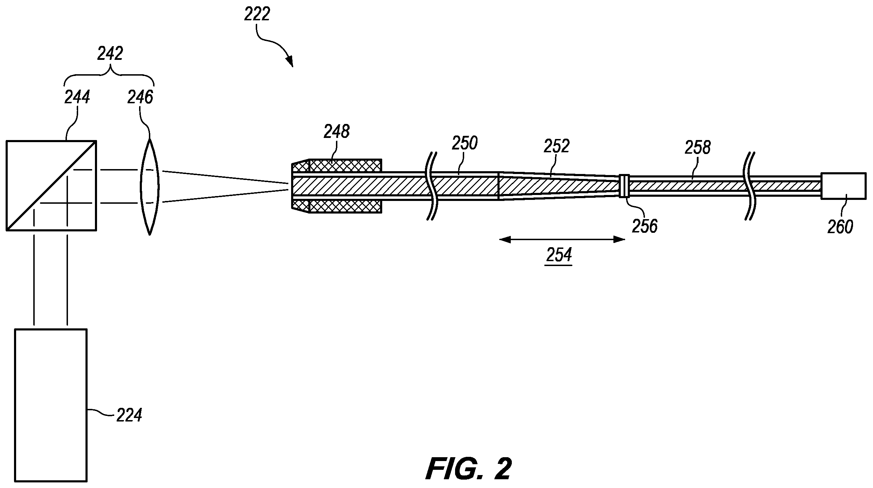

Figure 2 is a schematic cross-sectional view of one embodiment of the optical fiber 222, a coupling optics 242, and an energy source (in the embodiment shown in Figure 2, the energy source is a laser 224) for use within the catheter system 100. The laser 224 can generate an energy pulse that is directed towards the coupling optics 246. The laser 224 can be a pulsed IR laser or any suitable laser. In various embodiments, the catheter system 100 can include one or more emitter(s) 260 distributed along an active length(s) of the calcified vascular lesion(s) located at the treatment site 106.

The energy source described herein can be any suitable energy source for use within the catheter system 100. In some embodiments, the optical fiber 222 can be substituted with any suitable light carrier configured to receive an energy pulse. The optical fiber 222 can receive the energy pulse and can direct the energy pulse toward the emitter 260.

The coupling optics 242 couple and redirect the energy pulse toward the optical fiber 222. The coupling optics 242 can vary depending on the design requirements of the catheter system 100, the optical fiber 222, and/or the laser 224. It is understood that the coupling optics 242 can include additional systems, subsystems, components, and elements than those specifically shown and/or described herein. Additionally, or alternatively, the coupling optics 242 can omit one or more of the systems, subsystems, and elements that are specifically shown and/or described herein. The coupling optics 242 can include a reflector 244 and a lens 246. The reflector 244 can reflect the energy pulse towards the lens 246. The lens 246 can focus the energy pulse so that the optical fiber 222 is capable of receiving the energy pulse of proper diameter and/or size.

In the embodiment illustrated in Figure 2, the optical fiber 222 can include a ferrule 248, a first fiber member 250, a tapered portion 252 having a tapered portion length 254, a fused region 256, a second fiber member 258, and an emitter 260.

The ferrule 248 can organize and align the light carrier (e.g., the optical fiber 222) to the coupling optics 242 in the multiplexer (not shown). The ferrule 248 can vary depending on the design requirements of the catheter system 100, the optical fiber 222, and/or the laser 224. It is understood that the ferrule 248 can include additional systems, subsystems, components, and elements than those specifically shown and/or described herein. Additionally, or alternatively, the ferrule 248 can omit one or more of the systems, subsystems, and elements that are specifically shown and/or described herein.

The ferrule 248 can be a rigid member configured to confine and support the optical fiber. The ferrule 248 can also serve as a connector and/or a mechanical splice in the catheter system 199. The ferrule 248 can substantially surround any portion of the optical fiber 222.

In certain embodiments, the system described herein can include a segmented light carrier (e.g., the optical fiber 222) that is larger at its fiber proximal end and reduces to a smaller diameter towards the fiber distal end. Alternatively, the light carrier can be larger at the fiber distal end and reduce to a smaller diameter at the fiber proximal end.

The distal section (e.g., the second fiber member 258) can consist of a single light carrier or a plurality of light carriers leading to the emitter(s) 260. The proximal section (e.g., the first fiber member 250) can also consist of a single light carrier or a plurality of light carriers leading to the coupling optics 242. The tapered light carrier can improve pressure wave generation and the catheter system 100 performance.

The first fiber member 250 can be configured to be fixed to the ferrule 248. The first fiber member 250 can be a segment of the optical fiber 222 that is proximal to the laser 224 and the coupling optics 242. The first fiber member 250 can vary depending on the design requirements of the catheter system 100, the optical fiber 222, and/or the laser 224. It is understood that the first fiber member 250 can include additional systems, subsystems, components, and elements than those specifically shown and/or described herein. Additionally, or alternatively, the first fiber member 250 can omit one or more of the systems, subsystems, and elements that are specifically shown and/or described herein.

In certain embodiments, the first fiber member 250 can include the first tapered portion 252. In other embodiments, the first fiber member 250 can be separately attached or coupled to the tapered portion 252. The first fiber member 250 can be tapered and/or otherwise varied to have a modified shape. The first fiber member 250 can be a separate fiber or fiber member that is attached to the tapered portion 252 and/or the second fiber member 258.

The tapered portion 252 can allow for the optical fiber 222 to have a reduced diameter at the distal portion of the optical fiber 222 (e.g., the second fiber member 258). The tapered portion 252 can vary depending on the design requirements of the catheter system 100, the optical fiber 222, and/or the laser 224. The shape, taper, and/or dimensions of the tapered portion 252 can also vary. The tapered portion 252 can have a tapered portion length 254. The tapered portion length 254 can have varying lengths depending on the design requirements of the catheter system 100 and/or the optical fiber.

The location of the tapered portion 252 on the optical fiber 222 can vary. In some embodiments, the tapered portion 252 is located in close proximity to the coupling optics 242. In other embodiments, the tapered portion 252 is located closer to the balloon 104. It is appreciated that the tapered portion 252 can be located at any location along the optical fiber 222. In some embodiments, the optical fiber 222 can include a plurality tapered portions 252, located along varying locations of the optical fiber 222. It is appreciated that the optical fiber 222 can include any number of tapered portions 252 to meet the design requirements of the catheter system 100 and/or optical fiber 222.

The fused region 256 can be formed by fusing the first fiber member 250 with the second fiber member 258. The fused region 256 can join fibers and/or fiber members of different diameters together. In some embodiments, the fused region 256 can be fused around a split and/or spliced portion (shown in Figures 3AA and 3BB) of the optical fiber 222, so that the two severed portions of the optical fiber 222 can be joined.

The fused region 256 can vary depending on the design requirements of the catheter system 100, the optical fiber 222, and/or the laser 224. The shape and/or dimensions of fused region 256 can also vary. The location of the fused region 256 on the optical fiber 222 can vary. In some embodiments, the fused region 256 is located in close proximity to the coupling optics 242. In other embodiments, the fused region 256 is located closer to the balloon 104. It is appreciated that the fused region 256 can be located in any location along the optical fiber 222.

The second fiber member 258 can be substantially similar to the first fiber member 250. However, in certain embodiments, the second fiber member 258 can have a smaller diameter than the first fiber member 250. The second fiber member 258 can be a segment of the optical fiber 222 that is distal to the laser 224 and the coupling optics 242. The second fiber member 258 can be in optical communication with the emitter 260.

The second fiber member 258 can vary depending on the design requirements of the catheter system 100, the optical fiber 222, the first fiber member 250, the tapered portion 252, and/or the emitter 260. It is understood that the second fiber member 258 can include additional systems, subsystems, components, and elements than those specifically shown and/or described herein. Additionally, or alternatively, the second fiber member 258 can omit one or more of the systems, subsystems, and elements that are specifically shown and/or described herein.

While the first fiber member 250 and second fiber member 258 are described herein, it is appreciated that the optical fiber 222 can include any number of fiber members. In some embodiments, the optical fiber 222 includes up to fifty fiber members. In other embodiments, the first fiber member 250 having a first tapered portion (not shown) can be fused to a second fiber member 258 having a second tapered portion (not shown) that can be fused to the third fiber member (not shown. The optical fiber 222 can include any number of fiber members that can each have any number of tapered portions and can each include any number of fused sections and/or other attachment points as known in the art.

The emitter 260 can be driven by the energy source. The emitter 260 can be a plasma generator. The optical fiber 222 can include one or more emitters 260. The emitter 260 can be located at any position along the portion of the optical fiber 222 located inside the balloon 104. The emitter 260 can produce one or more plasma pulses 134.

The emitter 260 can vary depending on the design requirements of the catheter system 100, the optical fiber 222, and/or the laser 224. It is understood that the emitter 260 can include additional systems, subsystems, components, and elements than those specifically shown and/or described herein. Additionally, or alternatively, the emitter 260 can omit one or more of the systems, subsystems, and elements that are specifically shown and/or described herein.

One method for tapering the light carrier (e.g., tapering the optical fiber 222) includes tapering the larger proximal section down by heating, drawing, and then cleaving it at the smaller end. The light carrier assembly can be formed by fusing the smaller diameter end to a smaller diameter carrier for the distal section. The light carrier can be fused using a fused joint.

The tapering method can include heating and drawing the proximal section continuously to make a longer light carrier with a smaller diameter for the distal section. The tapering method can include creating a short, tapered portion 252 and fusing the large end and small end to matching diameter light carriers. This embodiment can require two fused joints.

In certain embodiments, the light carrier (e.g., the optical fiber 222) leading to the laser-driven pressure wave generating device (e.g., the emitter 260) can include three sub-sections. The proximal section can use a light carrier with a larger core diameter than the distal section. The proximal section of the light carrier begins at diameter $D_p$ and can be tapered or joined to a tapered section that reduces the diameter to match the distal section with diameter $D_d$. The light energy can be coupled into the end face of the proximal section.

The large diameter of the proximal face can reduce the precision required in forming the coupled light energy beam and aligning the coupling optics 242 to the light guide (e.g., the optical fiber 222). This design can reduce the multiplexer accuracy requirements and the optomechanical tolerances for connectorizing the light carrier. The tapered portion 252 can preserve the energy pulse and transmits the energy pulse to the smaller diameter carrier with minimal losses.

In certain embodiments, etendue is conserved in the light guide. Etendue conservation mandates that the product of numerical aperture (NA) and diameter remain constant. As a result, the NA of the distal section of the taper must increase so that $NA_d = (D_p/D_d) NA_p$. For example, an optical fiber 222 with an intrinsic NA = 0.22 that is tapered from 200 microns to 100 microns increases the NA to NA = 0.44. If the smaller diameter end of the taper were fused to a light guide with similar NA = 0.22, the mismatch would cause significant coupling losses into the cladding of the smaller fiber.

In various embodiments, the optical fiber 222 can be tapered from 220 microns to 100 microns. The optical fiber 222 described herein can be tapered from an initial diameter of 100μm, 110μm, 120μm, 130μm, 140μm, 150μm, 160μm, 170μm, 180μm, 190μm, 200μm, 220μm, 240μm, 260μm, 280μm, 300μm, 320μm, 340μm, 360μm, 380μm, 400μm, 420μm, 440μm, 460μm, 480μm, 500μm, 520μm, 540μm, 560μm, 580μm, 600μm, 620μm, 740μm, 760μm, 780μm, 800μm, 850μm, 900μm, 1mm or greater than 1mm to a final diameter of less than 1μm, 1μm, 5μm, 10μm, 20μm, 30μm, 40μm, 50μm, 60μm, 70μm, 80μm, 90μm, 100μm, 110μm, 120μm, 130μm, 140μm, 150μm, 160μm, 170μm, 180μm, 190μm, 200μm, 220μm, 240μm, 260μm, 280μm, 300μm, 320μm, 340μm, 360μm, 380μm, 400μm, 420μm, 440μm, 460μm, 480μm, 500μm, 520μm, 540μm, 560μm, 580μm, 600μm, 620μm, 740μm, 760μm, 780μm, 800μm, 850μm, 900μm. It is appreciated that the optical fibers and/or light carriers illustrated and/or described herein can be tapered to have final diameters and initial diameters that can fall within a range, wherein any of the foregoing numbers can serve as the lower or upper bound of the range, provided that the lower bound of the range is a value less than the upper bound of the range. The optical fibers and/or light carriers described herein can have final diameters and initial diameters that fall outside of the range described herein.

One method to avoid the coupling losses is to use a light carrier with lower NA on the fiber proximal end having the larger diameter and a light carrier with NA greater by the ratio of diameters for the smaller distal section. The tapering process from $D_p$ down to $D_d$ matches the numerical apertures of the two sections. In some embodiments, the tapered portion 252 can be formed to produce an adiabatic change in light bending and resulting NA to minimize losses through the transition.

Stimulated Brillouin Scattering (SBS) is a non-linear process in the light carrier that can limit total energy transmission. In SBS, an intense beam of light energy can interact with an acoustic wave in the medium, leading to a weak reflected beam. The reflected beam is generally referred to as a Stokes wave, and the original beam can then interfere to amplify the acoustic wave through electrostriction. The power in the reflected beam can increase nonlinearly with input beam power. As a result, energy throughput can be continually reduced with input power, effectively clamping the peak transmitted power which significantly limits the conversion efficiency and yield of the emitter.

SBS depends on many variables including the wavelength and bandwidth of the input light energy, physical properties of the light carrier medium, and its mechanical dimensions. In general, increasing the diameter of a light guide by a factor of two increases the power threshold for the onset of SBS by a factor of four. SBS originates in a region of the light guide closer to the launch end. If this can be avoided in the larger carrier then the full power launched into it can be coupled into the smaller light carrier further down. The larger diameter of the proximal section allows significantly more power to be coupled to the light guide assembly and transmit to the fiber distal end.

In some embodiments, the proximal light carrier can be a doped optical fiber that improves transmission characteristics and further suppresses the onset of SBS. Some examples of suitable dopants are $GeO_2$, $P_2O_3$, $TiO_2$, $B_2O_3$, $F_2$, $Al_2O_3$. Doped fibers can be much more expensive per unit length than conventional, pure silica fibers.

This approach can allow a short length of an expensive fiber to be used to suppress SBS with a much longer assembly made of lower-cost fiber. Other approaches could implement a short length of photonic crystal fiber, hollow-core fiber, chiral fibers, or similar structured optical fiber in the proximal section and conventional pure fused silica fiber through the longer length of the catheter system 100. This configuration can allow a short length of more expensive fiber to be used in the proximal section with lower costs for the distal section, achieving similar results.

Figure 3A is a schematic cross-sectional view of one embodiment of an optical fiber 322 for use within the catheter system 100. As shown in Figure 3A the optical fiber 322 can include a first fiber member 350, a fused region 356, a second fiber member 358 including second fiber legs 362A-C. The optical fiber 322 can include any number of second fiber legs 362.

The optical fiber 322 can include a single proximal section joined to a plurality of distal sections. In this embodiment, three distal carriers (e.g., second fiber legs 362A-C) can be fused to a single proximal carrier. The distal carriers can have the same diameter as that used in the proximal section or they can have smaller diameters. In other embodiments, the distal section comprises N light carriers with smaller diameters than the proximal section.

The transmission coupling efficiency can be maximized when the combined end face area of the distal section equals that of the proximal section, $D_d = D_p/\sqrt{N}$ . As a result, a 200/220 μm core/cladding light carrier matches well with three 105/125 μm carriers as illustrated in Figure 3A. The transmission coupling efficiency can be further optimized by thinning the cladding (not shown) on the distal carriers near the fused region 356 to bring the cores closer together and better match with the core of the proximal section. Compressing the region where the cores are joined during fusing can improve overlap.

One advantage of the embodiment illustrated in Figure 3A is that it eliminates the need to split the energy source into two separate beams and couple each of the beams into separate light carriers when powering two emitters 260 simultaneously. This embodiment can have another advantage of simplifying multiplexer architecture and optics. The embodiment shown in Figure 3A can also reduce the losses from having two separate beam paths and separate Fresnel losses occurring at the end face of two light carriers.

While there may be sizeable losses in a fused splitter (e.g., the fused region 356), these losses can be comparable to losses in a two-leg multiplexer architecture with complex coupling optics. The fused splitter can simplify multiplexer architecture, can reduce the alignment tolerances allowing simpler optics with improved transmission, and can eliminate losses due to splitting optics and the launch interface.

Figure 3B is a schematic cross-sectional view of another embodiment of another optical fiber 322 for use within the catheter system 100. The embodiment shown in Figure 3B illustrates a highly optimized 1x2 splitter that transitions from a 200 μm core proximal section to two 105 μm core distal sections. The transition between sections can be optimized by tapering the proximal light carrier to have an area equal to two times that of a 105 μm carrier. The two distal carriers can be formed to better match up with the fiber proximal end.

A separate capillary 364 can be placed around the fused assemblage (e.g., the fused region 356) and fused to optimize the cross-section and the area matching. This approach can be used to improve coupling efficiency for any number of carriers in the splitter. The capillary 364 can be drawn to tight tolerances and can be suitable for use in the catheter system 100. The capillary 364 can vary depending on the design requirements of the catheter system 100 and/or the optical fiber 322. It is understood that the capillary 364 can include additional systems, subsystems, components, and elements than those specifically shown and/or described herein. Additionally, or alternatively, the capillary 364 can omit one or more of the systems, subsystems, and elements that are specifically shown and/or described herein.

Figure 3AA is a cross-sectional view of the optical fiber taken on line 3AA-3AA in Figure 3A. As illustrated in Figure 3AA, the cross-sectional view demonstrates that the fused region 356 can include the first fiber member 350 and the second fiber member legs 362A-C. The first fiber member 350 can be fused to the second fiber member legs 362A-C at different locations so that portions of the outer perimeter of the first fiber member 350 is fused to portions the outer perimeter of the second fiber leg 362A, the second fiber leg 362B, and the second fiber leg 362C.

Figure 3BB is a cross-sectional view of the optical fiber taken on line 3BB-3BB in Figure 3B. As illustrated in Figure 3BB, the cross-sectional view demonstrates that the fused region 356 can include the first fiber member 350 and the second fiber member legs 362A-B. The fused region 356 can be substantially surrounded by the capillary 364.

Figure 4A is a schematic cross-sectional view of another embodiment of the optical fiber 422A, the coupling optics 442A (e.g., a reflector 444A and a lens 446A), and the energy source (e.g., a laser 424A) for use within the catheter system 100 (illustrated in Figure 1). In some embodiments, the optical fiber 422A can include a ferrule 448A, a first fiber member 450A, a fused region 456A, an emitter 460A, and an endcap 466A.

The endcap 466A can provide a larger surface area to spread the converging incident beam over before it focuses down the energy source to couple into the light carrier. The shape, configuration and size of the endcap 466A can vary depending on the design requirements of the catheter system 100 and/or the optical fiber 422A. In one embodiment, the endcap 466A can have a substantially cylindrical configuration. Alternatively, the endcap 466A can have another suitable configuration.

The embodiment shown in Figure 4A illustrates a direct approach to improving coupling to the smaller light carriers using the endcap 466A fused to the proximal end. In general, the damage threshold for optical material is much lower at an air interface than within the bulk material, sometimes by an order of magnitude.

This approach can reduce peak irradiance at the surface, thereby increasing the damage threshold for the light carrier assembly. The damage threshold near the end face of the light carrier is close to the bulk threshold for the formation materials. This embodiment allows a significantly greater amount of energy to be coupled into a small diameter light carrier. The converging incident beam can be tightly centered on the endcap 466A and can be tightly aligned within the ferrule 448A to achieve alignment for optimal coupling. In certain embodiments, the tight alignment is necessary to center the converging incident beam on the endcap 466A.

Figure 4B is a schematic cross-sectional view of another embodiment of the optical fiber 422B, the coupling optics 442B, and the energy source (e.g., the laser 424B) for use within the catheter system 100 (illustrated in Figure 1). The embodiment illustrated in Figure 4B features a tapered assembly. The tapered light carrier can be a short section (e.g., the tapered portion 452B) confined within the ferrule 456B. This approach can provide a large end face for defocusing the incident beam and coupling high energies thereby increasing the assembly damage threshold. Simultaneously, the embodiment of Figure 4B can reduce the demands of aligning with the smaller light carrier through the optical guiding of energy through the tapered portion 452B.

The present invention may also be used in methods for treating a treatment site 106 (illustrated in Figure 1) within or adjacent to a vessel wall or heart valve, with such methods utilizing the devices disclosed herein.

Fibers

The optical fibers suitable for use herein can include various types of fibers suitable for optical communication with an energy source, such as lasers and lamps. In some embodiments, optical fibers can include fiber connectors and fiber tapers. Fiber tapers can be packaged with a solid metal tube in tens of millimeter length. To convert light from a large core fiber into a small core fiber, fiber tapers that taper a larger core fiber into a smaller core for splicing can be an effective method to minimize the insertion loss. In certain embodiments, fiber tapers can be integrated into fiber connectors and can form a new type of fiber device called a tapered fiber connector.

The fiber connectors can be configured to couple lights from one fiber to another fiber. The fiber connectors can include polished ferrules. The fibers can be encircled into the fiber connectors with epoxy. There are tens of different styles of fiber connectors, including ferrules with an outer diameter of 1.25mm LC and ones with 2.5mm SC. The inner diameter of a ferrule depends on the fiber sizes, sometimes between 126-127 $\mu$m diameter for single-mode fibers.

In some embodiments, the ferrules described herein can have diameters (both inner diameters and outer diameters) of greater than or equal to 1$\mu$m, 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 110$\mu$m, 120$\mu$m, 130$\mu$m, 140$\mu$m, 150$\mu$m, 160$\mu$m, 170$\mu$m, 180$\mu$m, 190$\mu$m, 200$\mu$m, 220$\mu$m, 240$\mu$m, 260$\mu$m, 280$\mu$m, 300$\mu$m, 320$\mu$m, 340$\mu$m, 360$\mu$m, 380$\mu$m, 400$\mu$m, 420$\mu$m, 440$\mu$m, 460$\mu$m, 480$\mu$m, 500$\mu$m, 520$\mu$m, 540$\mu$m, 560$\mu$m, 580$\mu$m, 600$\mu$m, 620$\mu$m, 740$\mu$m, 760$\mu$m, 780$\mu$m, 800$\mu$m, 850$\mu$m, 900$\mu$m, 1mm, 1.5mm, 2.0mm, 2.5mm, 3.0mm, 3.5mm, 4.0mm or greater than 4.0mm. It is appreciated that the ferrules illustrated and/or described herein can have diameters (both inner diameters and outer diameters) that can fall within a range, wherein any of the foregoing numbers can serve as the lower or upper bound of the range, provided that the lower bound of the range is a value less than the upper bound of the range. The ferrules described herein can have diameters (both inner diameters and outer diameters) that fall outside of the range described herein.

For high power laser fiber coupling, a large core diameter fiber can be used to reduce the power density at the fiber coupling surface to enhance the damage threshold. However, a large diameter fiber is not flexible for many applications, such as catheters for medical treatments. In these applications, converting light power from a large diameter fiber into a small diameter fiber is important. Connecting or splicing a large core fiber with a small core fiber can directly result in unacceptable insertion loss.

One embodiment of a method for converting light power includes tapering the larger core diameter fibers to match the smaller core diameter fiber and then splicing the fiber tapers to the small core fibers. However, fiber tapers and splice joints need to be well packaged, usually 3 mm diameter and 50 mm long metal tubing, which is extremely inconvenient in many applications.

By integrating fiber connectors with fiber tapers a new type of fiber connector is created, a tapered fiber connector. This type of fiber connector is regular in size, but a fiber taper is encircled with epoxy. Any suitable type of fiber connectors including all single-mode fiber connectors can be improved into tapered fiber connectors for receiving lights as the transverse offset will not introduce any significant loss.

In various embodiments, fusion splicers can taper fibers and splice the fiber tapers onto small core fibers. In other embodiments, other devices and methods can be used to taper fibers and splice the fiber tapers onto small core fibers. Fibers can be tapered using a hydrogen flame to heat a mid-section of fiber that is held under tension. Fibers can be tapered using known devices and methods within the art.

The fiber tapers and splice joints can be encircled in fiber ferrules with epoxy for polishing. Epoxies that can be used herein include thermal epoxies, room temperature cure epoxies (such as ND353), UV curing epoxies (such as the LOCTITE® 4310 UV adhesive), and other suitable epoxies known within the art. The processing can be simple and low cost and the fiber connectors that are integrated with fiber tapers can be configured for high power laser coupling. One type of fiber connector that can be used is a fiber end-cap for high power laser input and output with reduced power density on fiber surfaces. Fiber end-caps can require accurate alignment as end-cap glass is equivalent to a free space rather than a waveguide. However, fiber tapers can still function as waveguides and thus alignment is not necessary.

In some embodiments, when core/cladding diameters of 200/220 $\mu$m fibers are selected to couple high power laser and core/cladding diameters of 105/125 $\mu$m fibers are used to build catheters for medical treatments, the process of converting laser power from the large core diameter fibers to the small core fibers is important.

For example, when directly splicing core/cladding diameters of 200/220 $\mu$m larger fibers onto core/cladding 105/125 $\mu$m smaller fibers together, a large portion of the light will not couple from the large fiber into the small fiber, which results in a high insertion loss. As an estimation, the ratio of two core overlapping is:

$$\left(\frac{105}{200}\right)^2 = 0.275 \qquad\qquad \text{[Equation 1]}$$

which is about a 6dB loss. This insertion loss is even higher for higher-order modes as the higher-order modes are likely distributed far from the core central area. The configuration of step-index fibers is shown below, the refractive index of the fiber core $n_1$ is slightly larger than the cladding $n_2$. To form a total internal reflection for a light's propagation along the fiber, the maximum incident angle $\alpha$ must meet the following conditions:

$$\begin{cases} sin(\alpha) = n1 \cdot sin(\beta) \\ n1 \cdot cos(\beta) = n2 \end{cases}$$ [Equation 2]

Simplifying Equations 1 and 2:

$$sin(\alpha) = \sqrt{n_1{}^2 - n_2{}^2} = NA$$ [Equation 3]

*NA* is the numerical aperture of the fibers, for 0.22 numerical aperture, the maximum incident angle $\alpha$ is 12.7 degrees. For a non-tapered fiber, the angles of light remain unchanged along the fiber and emit at the same angles $\alpha$. However, for a tapered fiber, the emitting angle will be larger than the incident angle $\alpha$, depending on the tapering angle and length.

When the incident angle of light is small enough, or the light beam effective NA is significantly smaller than the small fiber $NA_2$, with a certain tapering angle and tapering length, it is possible that the effective NA of the emitting lights from the fiber taper is still within the smaller fiber acceptable angle range of *arcsin*($NA_2$), 12.7 degrees for 0.22 numerical aperture. The tapering angle $\delta$ is expressed as:

$$\delta = arctan\left(\frac{D_1 - D_2}{2L}\right) \approx \frac{D_1 - D_2}{2L}$$ [Equation 4]

the lights get $2\delta$ angle to increase at every reflection due to the tapered surface. For tapering core diameter of 200 $\mu$m large fiber going into a 105 $\mu$m small core with a 4 mm taper length makes the tapering angle $\delta$ = 0.68 degree. For tapering single-mode fibers, in order to keep the fundamental mode in the fiber virtually unchanged, the adiabatic tapers require the tapering angle to be small enough 0.35 degrees for SMF28. For multimode power coupling, the tapering angle could be larger, but it can keep lights from leaking into the cladding.

In certain embodiments, when a 3 mm diameter parallel free-space laser beam is focused by a 50 mm focal lens and coupled to a fiber taper, the NA of the incident beam is:

$$NA = \arctan\left(\frac{3}{2 \times 50}\right) = 0.03 \sim 1.72\,°$$ [Equation 5]

The light angles increase by $2\delta$ for every reflection at the surface of fiber tapers, thus the pitches between two reflections along the fiber become shorter and shorter while the angles become larger and larger. In order to prevent light leaking from the fiber core to the cladding, the largest angle of lights must be not more than *arcsin*($NA_2$).

When a 3mm diameter free space laser beam is focused by a lens with 50 mm focal length, tapering a 200 $\mu$m large diameter fiber into a small core 105 $\mu$m (fiber taper 200/105 $\mu$m), the fiber taper can convert all lights from the large core fiber into 105 $\mu$m small core fiber with no light leakage to the fiber cladding at the tapering area and the splice joint.

For tapering large diameters of 200$\mu$m, 300$\mu$m, and 400$\mu$m into a 105$\mu$m small core with a 5mm taper length, simulations show all light from larger core diameter fibers can be fully coupled to the small core fibers like an optical funnel. For fiber tapers of 1000/105$\mu$m and a 5 mm taper length, simulations show light leakage from the fiber core to the cladding, which results in a significant insertion loss as shown below. For fiber tapers of 200/105$\mu$m, simulations also show that the light-emitting angle with respect to the taper length is curved. The smaller angles or beam sizes are usually desired and thus the optimized taper length is at least 4~5mm.

In some embodiments, 500 $\mu$m core diameters can be tapered into a 105 $\mu$m small core for light conversion. However, simulations show that the tapering length must be longer than 5mm to avoid lights leaking into the cladding. The taper length can be 15 mm long for achieving a smaller emitting angle.

A fiber fusion splicer FSM100P can taper fibers from up to 500$\mu$m large fibers into a small size. Comparing with a 105/125$\mu$m fiber for high power light coupling, large-diameter fibers can significantly reduce the power density. For tapered connectors with fiber tapers with diameters of 200$\mu$m, 300, 400$\mu$m, and 500$\mu$m, the power densities can be reduced by ~4, 8, 15, and 22 times, respectively. Fusing fiber for tapering can expand the fiber core slightly, fiber core diameters can be tapered to be slightly smaller, e.g. taper 200 $\mu$m core diameter to ~95$\mu$m core for matching 105/125 $\mu$m fibers.

In some embodiments, the optical fibers and/or light carriers described herein can have diameters of greater than or equal to 11$\mu$m, 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 110$\mu$m, 120$\mu$m, 130$\mu$m, 140$\mu$m, 150$\mu$m, 160$\mu$m, 170$\mu$m, 180$\mu$m, 190$\mu$m, 200$\mu$m, 220$\mu$m, 240$\mu$m, 260$\mu$m, 280$\mu$m, 300$\mu$m, 320$\mu$m, 340$\mu$m, 360$\mu$m, 380$\mu$m, 400$\mu$m, 420$\mu$m, 440$\mu$m, 460$\mu$m, 480$\mu$m, 500$\mu$m, 520$\mu$m, 540$\mu$m, 560$\mu$m, 580$\mu$m, 600$\mu$m, 620$\mu$m, 740$\mu$m, 760$\mu$m, 780$\mu$m, 800$\mu$m, 850$\mu$m, 900$\mu$m, 1mm, 1.5mm, 2.0mm, 2.5mm, 3.0mm or greater than 3.0mm. It is appreciated that the optical fibers and/or light carriers illustrated and/or described herein can have diameters that can fall within a range, wherein any of the foregoing numbers can serve as the lower or upper bound of the range, provided that the lower bound of the range is a value less than the upper bound of the range. The optical fibers and/or light carriers described herein can have diameters that fall outside of the range described herein.

In various embodiments, the optical fibers and/or light carriers described herein can have taper lengths of greater than or equal to 0.5mm, 1.0mm, 1.5mm, 2.0mm, 2.5mm, 3.0mm, 3.5mm, 4.0mm, 4.5mm, 5.0mm, 5.5mm, 6.0mm, 6.5mm, 7.0mm, 7.5mm, 8.0mm, 8.5mm, 9.0mm, 9.5mm, 10.0mm, 11.0mm, 12.0mm, 13.0mm, 14.0mm, 15.0mm, 20mm, 25mm, 30mm or greater than 30mm. It is appreciated that the optical fibers and/or light carriers illustrated and/or described herein can have taper lengths that can fall within a range, wherein any of the foregoing numbers can serve as the lower or upper bound of the range, provided that the lower bound of the range is a value less than the upper bound of the range. The optical fibers and/or light carriers described herein can have taper lengths that fall outside of the range described herein.

Lasers

The lasers suitable for use herein can include various types of lasers including lasers and lamps. Suitable lasers can include short pulse lasers on the sub-millisecond timescale. In some embodiments, the laser can include lasers on the nanosecond (ns) timescale. The lasers can also include short pulse lasers on the picosecond (ps), femtosecond (fs), and microsecond (us) timescales. It is appreciated that there are many combinations of laser wavelengths, pulse widths, and energy levels that can be employed to achieve plasma in the balloon fluid of the catheters illustrated and/or described herein. In various embodiments, the pulse widths can include those falling within a range including from at least 10 ns to 200 ns. In some embodiments, the pulse widths can include those falling within a range including from at least 20 ns to 100 ns. In other embodiments, the pulse widths can include those falling within a range including from at least 1 ns to 5000 ns.

Exemplary nanosecond lasers can include those within the UV to IR spectrum, spanning wavelengths of about 10 nanometers to 1 millimeter. In some embodiments, the lasers suitable for use in the catheter systems herein can include those capable of producing light at wavelengths of from at least 350 nm to 2000 nm. In some embodiments, the lasers can include those capable of producing light at wavelengths of from at least 700 nm to 3000 nm. In some embodiments, the lasers can include those capable of producing light at wavelengths of from at least 100 nm to 10 micrometers ($\mu$m). Nanosecond lasers can include those having repetition rates of up to 200 kHz. In some embodiments, the laser can include a Q-switched thulium:yttrium-aluminum-garnet (Tm:YAG) laser. In some embodiments, the laser can include a neodymium:yttrium-aluminum-garnet (Nd:YAG), holmium:yttrium-aluminum-garnet (Ho:YAG), erbium:yttrium-aluminum-garnet (Er:YAG), excimer laser, helium-neon laser, carbon dioxide laser, as well as doped, pulsed, fiber lasers.

Pressure Waves

The catheters illustrated and/or described herein can generate pressure waves having maximum pressures in the range of at least 1 megapascal (MPa) to 100 MPa. The maximum pressure generated by a particular catheter will depend on the laser, the absorbing material, the bubble expansion, the propagation medium, the balloon material, and other factors. In some embodiments, the catheters illustrated and/or described herein can generate pressure waves having maximum pressures in the range of at least 2 MPa to 50 MPa. In other embodiments, the catheters illustrated and/or described herein can generate pressure waves having maximum pressures in the range of at least 2 MPa to 30 MPa. In yet other embodiments, the catheters illustrated and/or described herein can generate pressure waves having maximum pressures in the range of at least 15 MPa to 25 MPa. In some embodiments, the catheters illustrated and/or described herein can generate pressure waves having peak pressures of greater than or equal to 1 MPa, 2 MPa, 3 MPa, 4 MPa, 5 MPa, 6 MPa, 7 MPa, 8 MPa, 9 MPa, 10 MPa, 11 MPa, 12 MPa, 13 MPa, 14 MPa, 15 MPa, 16 MPa, 17 MPa, 18 MPa, 19 MPa, 20 MPa, 21 MPa, 22 MPa, 23 MPa, 24 MPa, or 25 MPa, 26 MPa, 27 MPa, 28 MPa, 29 MPa, 30 MPa, 31 MPa, 32 MPa, 33 MPa, 34 MPa, 35 MPa, 36 MPa, 37 MPa, 38 MPa, 39 MPa, 40 MPa, 41 MPa, 42 MPa, 43 MPa, 44 MPa, 45 MPa, 46 MPa, 47 MPa, 48 MPa, 49 MPa, or 50 MPa. It is appreciated that the catheters illustrated and/or described herein can generate pressure waves having operating pressures or maximum pressures that can fall within a range, wherein any of the foregoing numbers can serve as the lower or upper bound of the range, provided that the lower bound of the range is a value less than the upper bound of the range.

Therapeutic treatment can act via a fatigue mechanism or a brute force mechanism. For a fatigue mechanism, operating pressures would be about at least 0.5 MPa to 2 MPa, or about 1 MPa. For a brute force mechanism, operating pressures would be about at least 20 MPa to 30 MPa, or about 25 MPa. Pressures between the extreme ends of these two ranges may act upon a treatment site using a combination of a fatigue mechanism and a brute force mechanism.

The pressure waves described herein can be imparted upon the treatment site from a distance within a range from at least 0.01 millimeters (mm) to 25 mm extending radially from a longitudinal axis of a catheter placed at a treatment site. In some embodiments, the pressure waves can be imparted upon the treatment site from a distance within a range from at least 1 mm to 20 mm extending radially from a longitudinal axis of a catheter placed at a treatment site. In other embodiments, the pressure waves can be imparted upon the treatment site from a distance within a range from at least 0.1 mm to 10 mm extending radially from a longitudinal axis of a catheter placed at a treatment site. In yet other embodiments, the pressure waves can be imparted upon the treatment site from a distance within a range from at least 1.5 mm to 4 mm extending radially from a longitudinal axis of a catheter placed at a treatment site. In some embodiments, the pressure waves can be imparted upon the treatment site from a range of at least 2 MPa to 30 MPa at a distance from 0.1 mm to 10 mm. In some embodiments, the pressure waves can be imparted upon the treatment site from a range of at least 2 MPa to 25 MPa at a distance from 0.1 mm to 10 mm. In some embodiments, the pressure waves can be imparted upon the treatment site from a distance that can be greater than or equal to 0.1 mm, 0.2 mm, 0.3 mm, 0.4 mm, 0.5 mm, 0.6 mm, 0.7 mm, 0.8 mm, or 0.9 mm, 1 mm, 2 mm, 3 mm, 4 mm, 5 mm, 6 mm, 7 mm, 8 mm, 9 mm, or 10 mm, or can be an amount falling within a range between, or outside the range of any of the foregoing.

The systems and methods described herein provide improved optical coupling to an individual light carrier that is organized in a multi-channel array. The advantage of the improved optical coupling is to increase the damage threshold and coupling efficiency while reducing demand on mechanical tolerances.

The systems and methods provided herein improve an optical damage threshold and energy throughput while maintaining a small diameter section leading to the plasma generator. The advantage of the improved optical damage threshold and energy throughput is to minimize the mechanical cross-section and bending stiffness of the light guide bundle and catheter.

The systems and methods provided herein allow the use of larger light carriers in the proximal section of the single-use device (sometimes referred to herein as a "SUD") that are easy to align optics with to couple light into while allowing smaller carriers to be used to in the distal section to optimize key mechanical characteristics such as crossing the profile and bending stiffness. Advantages of this approach are that it: 1) reduces demand on the connector tolerances for alignment of the light carrier to the coupling optics and mechanical tolerances of their location in a multi-channel array, 2) reduces system performance dependence on the accuracy of connecting and aligning the multi-channel array to the multiplexer, 3) reduces dependence on the accuracy of the positioning mechanism in the multiplexer and associated quality and precision of its optical and mechanical components, 4) makes it possible to use lower-cost, lower accuracy ferrules on the SUD, reducing costs, 5) increases optical damage threshold at the proximal end face of the light carrier allowing increased energy to drive the emitters, and 6) reduces transmission losses in the light carrier that are due to non-linear optical processes that increase with the decrease in light carrier diameter.

The systems and methods described herein reduce the optical coupling dependence on the precision and mechanical tolerance stack-ups of assemblies and true alignment for light carrier(s), ferrule(s), connector(s), and receptacle(s), thereby making it possible to use low-cost, low-precision components on the SUD and improve the cost of goods sold.

The systems and methods described herein reduce the multiplexer performance dependence on the accuracy of the positioning mechanism and the associated quality and precision of its optical and mechanical components, thereby improving the speed and performance of the multiplexer and the multi-channel ferrule system.

The systems and methods described herein increase the damage threshold of the light carrier(s) and allow higher energies to be coupled into the light carrier(s) and to the emitter(s) while enabling the use of smaller carrier(s) through the critical sections of the catheter.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

As used herein, the recitation of numerical ranges by endpoints shall include all numbers subsumed within that range, inclusive (e.g., 2 to 8 includes 2, 2.1, 2.8, 5.3, 7, 8, etc.).

It is recognized that the figures shown and described are not necessarily drawn to scale, and that they are provided for ease of reference and understanding, and for relative positioning of the structures.

The headings used herein are provided for consistency with suggestions under 37 CFR 1.77 or otherwise to provide organizational cues. These headings shall not be viewed to limit or characterize the invention(s) set out in any claims that may issue from this disclosure. As an example, a description of a technology in the "Background" is not an admission that technology is prior art to any invention(s) in this disclosure. Neither is the "Summary" or "Abstract" to be considered as a characterization of the invention(s) set forth in issued claims.

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the scope herein.

It is understood that although a number of different embodiments of the catheter systems have been illustrated and described herein, one or more features of any one embodiment can be combined with one or more features of one or more of the other embodiments, provided that such combination satisfies the intent of the present invention.

While a number of exemplary aspects and embodiments of the catheter systems have been discussed above, those of skill in the art will recognize certain modifications, permutations, additions, and sub-combinations thereof. It is therefore intended that the following appended claims and claims hereafter introduced are interpreted to include all such modifications, permutations, additions, and sub-combinations as are within their scope, and no limitations are intended to the details of construction or design herein shown.

**Claims**

1. A catheter system for treating a treatment site within or adjacent to a vessel wall or a heart valve, the catheter system comprising:

   an inflatable balloon (104);
   an optical fiber (222) having (i) a fiber proximal end, and (ii) a fiber distal end positioned within the inflatable balloon, the optical fiber being configured to receive an energy pulse so that the optical fiber emits light energy in a direction away from the optical fiber to generate a plasma pulse within the inflatable balloon, the optical fiber including a tapered portion (252) positioned between the fiber proximal end and the fiber distal end, the tapered portion being spaced apart from the fiber distal end; and
   an energy source (224) in optical communication with the fiber proximal end of the optical fiber.

2. The catheter system of claim 1 wherein the optical fiber includes a first fiber member and a second fiber member that is coupled to the first fiber member.

3. The catheter system of claim 2 wherein at least a portion of the second fiber member is positioned within the inflatable balloon.

4. The catheter system of any one of claims 2-3 wherein the first fiber member includes an endcap.

5. The catheter system of claim 1 wherein the first fiber member comprising the tapered portion (252) and the second fiber member are formed as a unitary structure.

6. The catheter system of any one of claims 2-4 wherein the first fiber member is fused to the second fiber member at a fused region.

7. The catheter system of claim 6 wherein the first fiber member has a first fiber member distal region that is positioned outside the inflatable balloon; and wherein the second fiber member has a second fiber member proximal region that is positioned outside the inflatable balloon.

8. The catheter system of claim 7 wherein the first fiber member distal region is fused to the second fiber member proximal region in the fused region.

9. The catheter system of any one of claims 6-8 wherein the fused region is outside of the inflatable balloon.

10. The catheter system of any one of claims 6-9 further comprising a ferrule that encircles the fused region.

11. The catheter system of claim 10 wherein the ferrule has an outer diameter of greater than or equal to $500\mu m$.

12. The catheter system of any one of claims 10-11 wherein the ferrule is formed by one of a plastic and a metal.

13. The catheter system of any one of claims 1-12 wherein the optical fiber is tapered from a diameter of at least $150\ \mu m$ to a diameter of less than $120\ \mu m$.

14. The catheter system of any one of claims 1-13 wherein the tapered portion has a tapered portion length of at least 4 mm.

15. The catheter system of any one of claims 1-14 wherein the energy source includes a laser.

**Patentansprüche**

1. Kathetersystem zur Behandlung einer Behandlungsstelle in oder benachbart zu einer Gefäßwand oder einer Herzklappe, wobei das Kathetersystem aufweist:

   einen aufblasbaren Ballon (104);
   eine optische Faser (222) mit (i) einem proximalen Faserende und (ii) einem distalen Faserende, das im aufblasbaren Ballon positioniert ist, wobei die optische Faser so konfiguriert ist, dass sie einen Energieimpuls empfängt, so dass die optische Faser Lichtenergie in einer von der optischen Faser wegführenden Richtung abstrahlt, um einen Plasmaimpuls im aufblasbaren Ballon zu erzeugen, die optische Faser einen zulaufenden Abschnitt (252) aufweist, der zwischen dem proximalen Faserende und dem distalen Faserende positioniert ist, und der zulaufende Abschnitt vom distalen Faserende beabstandet ist; und
   eine Energiequelle (224) in optischer Kommunikation mit dem proximalen Faserende der optischen Faser.

2. Kathetersystem nach Anspruch 1, wobei die optische Faser ein erstes Faserteil und ein zweites Faserteil aufweist, das mit dem ersten Faserteil gekoppelt ist.

3. Kathetersystem nach Anspruch 2, wobei zumindest ein Abschnitt des zweiten Faserteils im aufblasbaren Ballon positioniert ist.

4. Kathetersystem nach einem der Ansprüche 2 bis 3, wobei das erste Faserteil eine Endkappe aufweist.

5. Kathetersystem nach Anspruch 1, wobei das erste Faserteil mit dem zulaufenden Abschnitt (252) und das zweite Faserteil als einheitliche Struktur ausgebildet sind.

6. Kathetersystem nach einem der Ansprüche 2 bis 4, wobei das erste Faserteil mit dem zweiten Faserteil in einem verschweißten Bereich verschweißt ist.

7. Kathetersystem nach Anspruch 6, wobei das erste Faserteil einen ersten distalen Faserteilbereich hat, der außerhalb des aufblasbaren Ballons positioniert ist; und wobei das zweite Faserteil einen zweiten proximalen Faserteilbereich hat, der außerhalb des aufblasbaren Ballons positioniert ist.

8. Kathetersystem nach Anspruch 7, wobei der erste distale Faserteilbereich mit dem zweiten proximalen Faserteilbereich im verschweißten Bereich verschweißt ist.

9. Kathetersystem nach einem der Ansprüche 6 bis 8, wobei der verschweißte Bereich außerhalb des aufblasbaren Ballons liegt.

10. Kathetersystem nach einem der Ansprüche 6 bis 9, ferner mit einer Hülse, die den verschweißten Bereich umschließt.

11. Kathetersystem nach Anspruch 10, wobei die Hülse einen Außendurchmesser gleich oder größer als 500 $\mu$m hat.

12. Kathetersystem nach einem der Ansprüche 10 bis 11, wobei die Hülse durch einen Kunststoff oder ein Metall gebildet ist.

13. Kathetersystem nach einem der Ansprüche 1 bis 12, wobei die optische Faser von einem Durchmesser von mindestens 150 $\mu$m auf einen Durchmesser unter 120 $\mu$m zuläuft.

14. Kathetersystem nach einem der Ansprüche 1 bis 13, wobei der zulaufende Abschnitt eine Länge des zulaufenden Abschnitts von mindestens 4 mm hat.

15. Kathetersystem nach einem der Ansprüche 1 bis 14, wobei die Energiequelle einen Laser aufweist.

**Revendications**

1. Système de cathéter pour le traitement d'un site de traitement au sein de, ou adjacent à, une paroi de vaisseau ou une valvule cardiaque, le système de cathéter comprenant :

un ballonnet gonflable (104) ;
une fibre optique (222) ayant (i) une extrémité proximale de fibre, et (ii) une extrémité distale de fibre positionnée au sein du ballonnet gonflable, la fibre optique étant configurée pour recevoir une impulsion d'énergie de sorte que la fibre optique émette une énergie lumineuse dans une direction s'éloignant de la fibre optique pour générer une impulsion de plasma au sein du ballonnet gonflable, la fibre optique incluant une partie effilée (252) positionnée entre l'extrémité proximale de fibre et l'extrémité distale de fibre, la partie effilée étant espacée de l'extrémité distale de fibre ; et
une source d'énergie (224) en communication optique avec l'extrémité proximale de fibre de la fibre optique.

2. Système de cathéter selon la revendication 1 dans lequel la fibre optique inclut un premier élément de fibre et un deuxième élément de fibre qui est couplé au premier élément de fibre.

3. Système de cathéter selon la revendication 2 dans lequel au moins une partie du deuxième élément de fibre est positionnée au sein du ballonnet gonflable.

4. Système de cathéter selon l'une quelconque des revendications 2 à 3 dans lequel le premier élément de fibre inclut un embout.

5. Système de cathéter selon la revendication 1 dans lequel le premier élément de fibre comprenant la partie effilée (252) et le deuxième élément de fibre se présentent sous la forme d'une structure unitaire.

6. Système de cathéter selon l'une quelconque des revendications 2 à 4 dans lequel le premier élément de fibre est fusionné avec le deuxième élément de fibre au niveau d'une région fusionnée.

7. Système de cathéter selon la revendication 6 dans lequel le premier élément de fibre possède une région distale de premier élément de fibre qui est positionnée à l'extérieur du ballonnet gonflable ; et dans lequel le deuxième élément de fibre possède une région proximale de deuxième élément de fibre qui est positionnée à l'extérieur du ballonnet gonflable.

8. Système de cathéter selon la revendication 7 dans lequel la région distale de premier élément de fibre est fusionnée avec la région proximale de deuxième élément de fibre dans la région fusionnée.

9. Système de cathéter selon l'une quelconque des revendications 6 à 8 dans lequel la région fusionnée est à l'extérieur du ballonnet gonflable.

10. Système de cathéter selon l'une quelconque des revendications 6 à 9 comprenant en outre une virole qui encercle la région fusionnée.

11. Système de cathéter selon la revendication 10 dans lequel la virole possède un diamètre externe supérieur à ou égal à 500 μm.

12. Système de cathéter selon l'une quelconque des revendications 10 à 11 dans lequel la virole est formée de l'un parmi un plastique et un métal.

13. Système de cathéter selon l'une quelconque des revendications 1 à 12 dans lequel la fibre optique est effilée d'un diamètre d'au moins 150 μm à un diamètre de moins de 120 μm.

14. Système de cathéter selon l'une quelconque des revendications 1 à 13 dans lequel la partie effilée possède une longueur de partie effilée d'au moins 4 mm.

15. Système de cathéter selon l'une quelconque des revendications 1 à 14 dans lequel la source d'énergie inclut un laser.

**FIG. 1**

**FIG. 2**

**FIG. 3A**

362A

362C

358

3BB

3BB

356

352

354

364

322

350

*FIG. 3B*

356 —

362A

350

362B

362C

**FIG. 3AA**

356 —

364

350

362A

362B

**FIG. 3BB**

**FIG. 4A**

EP 4 291 125 B1

**FIG. 4B**

EP 4 291 125 B1

**REFERENCES CITED IN THE DESCRIPTION**

**Patent documents cited in the description**

- US 20200397230 A1